# EUROPÄISCHE PATENTSCHRIFT

(11) **EP 0 795 534 B1**
(45) Veröffentlichungstag und Bekanntmachung des Hinweises auf die Patenterteilung: **02.02.2000**
(21) Anmeldenummer: 97103520.9
(22) Anmeldetag: 04.03.1997
(51) Int. Cl.: C07C 51/41, C07C 51/50, C07C 59/19

(54) **Verfahren zur Herstellung von Calziumpyruvat und dessen Hydraten**
Process for preparing calcium pyruvate and its hydrates
Procédé de préparation du pyruvate de calcium ainsi que ses hydrates

(30) Priorität: 11.03.1996 AT 44896
(43) Veröffentlichungstag der Anmeldung: 17.09.1997
(73) Patentinhaber: DSM Fine Chemicals Austria GmbH, 4021 Linz (AT)
(72) Erfinder: Zimmermann, Curt, Dr., 4310 Mauthausen (AT); Weissenböck, Kurt, 4470 Enns (AT)
(74) Vertreter: Klostermann, Ingrid

(56) Entgegenhaltungen:
- FR-A- 1 465 432

## Beschreibung

Calziumpyruvat findet als Zusatz in der Nahrungsmittel- und Getränkeindustrie Verwendung und wird ausgehend von Brenztraubensäure hergestellt.

In FR-PS 1,465,432 ist zum Beispiel ein Verfahren beschrieben, bei welchem eine etwa 13 %ige wässrige Lösung von Brenztraubensäure mit Calziumcarbonat versetzt wird und das Reaktionsgemisch für einige Minuten erwärmt und anschließend abgekühlt wird, worauf ein Niederschlag ausfällt. Die überstehende Lösung wird sodann bei 95°C mit Ethanol versetzt, worauf, nach dem Abkühlen, Calziumpyruvat ausfällt, das abfiltriert und mit Ethanol gewaschen wird. Der Nachteil bei diesem Verfahren ist, wie ein Vergleichsversuch zeigte, daß das anfallende Produkt zum Großteil aus cyclischen Dimeren und Oligomeren der Brenztraubensäure besteht, wobei nach Abtrennung der Dimeren und Oligomeren das gewünschte Monomere nur in einer Ausbeute von wenigen Prozenten erhalten wird.

Aufgabe der vorliegenden Erfindung war es demnach, ein Verfahren zu finden, das, ausgehend von Brenztraubensäure, Calziumpyruvat auf einfache Weise und in hoher Reinheit, insbesondere mit einem sehr geringen Anteil von bis zu unter 0,1 % an Dimeren und Oligomeren liefert.
Unerwarteterweise konnte diese Aufgabe durch das erfindungsgemäße Verfahren gelöst werden.

Gegenstand der vorliegenden Erfindung ist demnach ein Verfahren zur Herstellung von hochreinem Calziumpyruvat oder dessen Hydrate, durch Umsetzen einer wässrigen Lösung von Brenztraubensäure mit einer anorganischen Calziumverbindung und unter Einstellung des pH-Wertes auf 2 bis 7, vorzugsweise 2,5 bis 4, das dadurch gekennzeichnet ist, daß eine bis zu 10 %ige wässrige Lösung von Brenztraubensäure gegebenenfalls in Gegenwart eines Stabilisators oder Komplexbildners mit der Calziumverbindung umgesetzt wird und die so erhaltene Lösung bei Temperaturen von 0 bis 30°C mit einem organischen Verdünnungsmittel vermischt wird und der entstandene Niederschlag an Calziumpyruvat oder dessen Hydrat aus der Lösung isoliert wird.

Bei dem erfindungsgemäßen Verfahren wird Brenztraubensäure in soviel Wasser gelöst, daß eine bis zu 10 %ige Lösung erhalten wird. Bevorzugt wird eine 1 bis 8 %ige, besonders bevorzugt eine 3 bis 6 %ige Lösung hergestellt. Zu dieser Lösung wird sodann vorzugsweise unter Rühren eine Calziumverbindung zugegeben.

Als Calziumverbindung eignen sich anorganische Calziumverbindungen, wie beispielsweise Calziumcarbonat, Calziumhydroxyd, usw. Bevorzugt wird Calziumcarbonat verwendet. Die Calziumverbindung wird dabei in einer äquivalenten Menge, d h. pro Mol Brenztraubensäure ein halbes Mol Calziumverbindung zugegeben. Bevorzugt wird jedoch ein leichter Überschuß an Brenztraubensäure eingesetzt.
Durch die Zugabe der Calziumverbindung stellt sich ein pH-Wert von etwa 2 bis 7, bevorzugt von 2,5 bis 4 ein.
Die Reaktionstemperatur liegt ungefähr bei etwa 0 °C bis 60°C, bevorzugt bei 10 bis 40°C, besonders bevorzugt bei 20 bis 30°C.
Zu Beginn der Reaktion kann gewünschtenfalls ein üblicher Komplexbildner wie etwa Ethylendiamintetraessigsäure (EDTA) oder dessen Salze zugesetzt werden, wodurch Schwermetallspuren wie etwa Eisenspuren, die beim Einsatz von Calziumsverbindungen, insbesondere von Calziumcarbonat eingebracht werden, maskiert werden.
Es kann auch eine organische Säure, bevorzugt eine organische Säure, die sich auch als Lebensmittelzusatz eignet, als Stabilisator eingesetzt werden, wodurch ein pH-Wert unter 7 während dem Herstellverfahren sichergestellt wird und zum Teil ebenfalls Schwermetallspuren maskiert werden. Geeignete Säuren sind beispielsweise Zitronensäure, Ascorbinsäure, Weinsäure, Äpfelsäure, Fumarsäure, Oxalsäure, usw.. Bevorzugt wird Zitronensäure als Zusatz verwendet.
Die Menge an zugesetztem Stabilisator bzw. Komplexbildner liegt bevorzugt zwischen 0,001 und 10 mol%, besonders bevorzugt bei 0,1 bis 5 mol% bezogen auf Brenztraubensäure.

Anschließend wird die so erhaltene klare, farblose Lösung mit einem organischen Verdünnungsmittel vermischt. Bevorzugt wird dabei ein niedrigsiedendes, mit Wasser gut mischbares, organisches Verdünnungsmittel verwendet. Beispiele für geeignete Verdünnungsmittel sind Ketone wie etwa Aceton, Ethylmethylketon usw., Alkohole, wie etwa Ethanol, Methanol usw., Ether wie etwa 1,4 Dioxan u.a. oder Ester u.a.. Bevorzugt wird Ethanol oder Aceton, besonders bevorzugt Aceton eingesetzt.
Die Reaktionstemperatur liegt dabei bei etwa 0 bis 30 °, bevorzugt bei etwa 5 bis 15°C.

Der dabei ausfallende Niederschlag an Calziumpyruvat wird sodann vom Verdünnungsmittel befreit, beispielsweise durch Abfiltrieren, Zentrifugieren, Dekantieren, anschließend vorzugsweise mit dem zum Ausfällen verwendeten Verdünnungsmittel gewaschen, wiederum von dem Verdünnungsmittel, etwa durch Zentrifugieren, Dekantieren, Absaugen usw. befreit. Das Feuchtprodukt wird sodann beispielsweise gefriergetrocknet oder in üblichen Trocknern bei Normaldruck oder im Vakuum getrocknet . Bevorzugt erfolgt die Trocknung im Vakuum. Die Temperaturen bei der Vakuumtrocknung liegen bevorzugt unter 100°C, besonders bevorzugt zwischen 30 und 60°C.
In Abhängigkeit von den Trockenbedingungen variiert der Wassergehalt des erhaltenen Calziumpyruvates.
Das so erhaltene Calziumpyruvat bzw. dessen Hydrate werden durch das erfindungsgemäße Verfahren in hohen Ausbeuten und in hoher Reinheit erhalten. Der Dimerenanteil liegt bei dem erfindungsgemäß hergestellten Calziumpyruvat-Monohydrat bis zu unter 0,1 % (HPLC), Trimere und höhrere Oligomere sind nicht mehr nachweisbar.

### Beispiel 1:

45 g (0,5 mol) Brenztraubensäure (98 %) wurden in 880 g VE-Wasser gelöst und unter Rühren innerhalb von 10 Min. mit 23 g (0,23 mol) Calziumcarbonat auf pH 3,5 gestellt. Die entstandene klare, farblose Lösung wurde innerhalb von 15 Min. unter Rühren mit 2000 ml kaltem Aceton vermischt. Der entstandene weiße Niederschlag wurde durch Zentrifugieren vom Lösungsmittel befreit, mit Aceton gewaschen und nochmals zentrifugiert. Das so erhaltne Produkt wies einen Dimeranteil von 0.1 % (HPLC, UV-Det. 204 nm) auf. Trimere oder höhere Oligomere bzw. Polymere waren nicht nachweisbar. Nach 4 h Trocknung im Vakuum bei 40°C wurden 52 g eines reinweißen Produktes mit einem Gehalt an Calziumpyruvat-Monohydrat von 98 % erhalten. Das entspricht einer Ausbeute von 88 % bezogen auf Brenztraubensäure.

### Vergleichsbeispiel: analog FR-PS 1,465,432

27 g (0,3 mol) Brenztraubensäure (98%) wurden in 200 g VE-Wasser gelöst und unter Rühren vorsichtig mit 16,5 g (0,165 mol) Calziumcarbonat versetzt. Es resultierte eine dicke kaum rührbare breiartige Masse, die beim Erwärmen in eine gelb-rötliche trübe Flüssigkeit übergeht. Nach dem Abkühlen bildete sich ein Niederschlag. Die überstehende Lösung wurde dekantiert und mit 600 ml Ethanol bei 95°C vermischt. Der dabei entstehende Niederschlag, wurde abgesaugt und mit 50 ml Ethanol gewaschen. Aus 71 g Feuchtprodukt wurden nach Trocknung im Vakuum 28 g eines Produktes ethalten, das nach HPLC-Analyse nur zu 4 Gew.% aus Calziumpyruvat bestand. Der Hauptbestandteil waren offene und cyclische Dimere und Oligomere der Brenztraubensäuresalze sowie Wasser.
18,4 g dieses Produktes wurden in 100 ml warmem Wasser gelöst und von geringen Mengen unlöslicher Anteile durch Filtration befreit. Nach Zugabe von Aceton fiel ein Niederschlag aus, der filtriert, mit Aceton gewaschen und im Vakuum getrocknet wurde. Die Analyse des erhaltenen Produktes ergab gegenüber dem nicht umkristallisierten Produkt einen noch niedrigeren Calziumpyruvat-Anteil und einen entsprechend hohen Oligomeranteil.

### Beispiel 2:

### Erhöhung der Stabilität und Produktqualität durch Zusetzen von Stabilisatoren

4,5 g (0,05 mol) Brenztraubensäure (98 %) wurden in 88 g VE-Wasser gelöst und mit 2 g (0,02 mol) Calziumcarbonat versetzt. Die so erhaltene klare Lösung wurde in 200 ml Aceton eingetropft. Der dabei entstandene weiße Niederschlag wurde mit Hilfe einer Glassinternutsche von der Mutterlauge getrennt, mit Aceton gewaschen und im Stickstoffstrom getrocknet.

Analog wurde die gleiche Menge Brenztraubensäure und 0,21 g Zitronensäure in 88 g VE-Wasser gelöst und wie oben weiterbehandelt.

Beide Produkte wurden bei Raumtemperatu gelagert und laufend analysiert. Der Verlauf der Nebenproduktbildung ist in nachfolgender Tabelle wiedergegeben.

| Ohne Zusätze | | mit Zintronensäurezusatz | |
|---|---|---|---|
| Lagerdauer in Tagen | Anteil an acyclischem Dimeren (%hplc) | Lagerdauer in Tagen | Anteil an acyclischem Dimeren (%hplc) |
| 0 | 0,9 | 0 | 0,3 |
| 1 | 1,2 | 1 | 0,4 |
| 5 | 2,1 | 6 | 0,7 |
| 8 | 2,4 | | |
| 12 | 2,8 | 14 | 1,1 |
| 19 | 3,1 | | |
| 27 | 3,9 | 27 | 1,5 |

### Beispiel 3:

### Erhöhung der Produktqualität durch Komplexierung von katalytisch aktiven Schwemetallspuren mit Hilfe von EDTA

16,8 g (0,05 mol) Brenztraubensäure (26,2 %) wurden in 75,6 g (VE-Wasser gelöst und mit 22 ml (0,02 mol) einer 10 % Suspension von Calziumcarbonat mit einem Eisenanteil von 260 ppm in Wasser versetzt. Es entstand eine klare, gelbliche Lösung. Nach 1,5 Stunden Rühren bei Raumtemperatur war die Calziumpyruvatlösung zu über 1 % dimerisiert. Sie wurde mit 200 ml kaltem Aceton versetzt. Der dabei entstandene weiße Niederschlag wurde mit einer Filterzentrifuge von der Mutterlauge getrennt. Das Feuchtprodukt wurde nach stehen lassen über Nacht bei 40°C im Vakuum (10 mbar) getrocknet. Es wurden 5.5 g eines leicht gelblichen Produktes erhalten. Der Gehalt an monomerem Calziumpyruvat x 2,5 H₂O war 90 % , der Anteil an acyclischem Dimer war über 4 %.

Analog wurde die gleiche Menge Brenztraubensäure gemeinsam mit 0,014 g Ethylendiamintetraessigsäure-Dinatriumsalz-Dihydrat in Wasser gelöst und mit der gleichen Menge an Calziumcarbonatsuspension in Wasser versetzt. Es entstand eine klare, farblose Lösung. nach 1,5 Stunden Rühren bei Raumtemperatur war die Calziumpyruvatlösung zu 0,7 % dimerisiert. Die Lösung wurde wie oben weiterbehandelt. Es wurden 5,5 g eines reinweißen Produktes erhalten. Der Gehalt an monomerem Calziumpyruvat x 2,5 H₂O war 97 %, der Anteil an acyclischem Dimer war 3 %.

## Patentansprüche

1. Verfahren zur Herstellung von hochreinem Calziumpyruvat oder dessen Hydrate, durch Umsetzen einer wässrigen Lösung von Brenztraubensäure mit einer anorganischen Calziumverbindung und unter Einstellung des pH-Wertes auf 2 bis 7, vorzugsweise 2,5 bis 4, dadurch gekennzeichnet, daß eine bis zu 10 %ige wässrige Lösung von Brenztraubensäure gegebenenfalls in Gegenwart eines Stabilisators oder Komplexbildners mit der Calziumverbindung umgesetzt wird und die so erhaltene Lösung bei Temperaturen von 0 bis 30°C mit einem organischen Verdünnungsmittel vermischt wird und der entstandene Niederschlag an Calziumpyruvat oder dessen Hydrat aus der Lösung isoliert wird.

2. Verfahren nach Anspruch 1, dadurch gekennzeichnet, daß eine 1 bis 8 %ige wässrige Lösung von Brenztraubensäure verwendet wird.

3. Verfahren nach Anspruch 1, dadurch gekennzeichnet, daß die Reaktion in Gegenwart eines Komplexbildners zur Maskierung von Verunreinigungen durch Schwermetallspuren durchgeführt wird.

4. Verfahren nach Anspruch 3, dadurch gekennzeichnet, daß als Komplexbildner Ethylendiamintetraessigsäure oder dessen Salze verwendet werden.

5. Verfahren nach Anspruch 1, dadurch gekennzeichnet, daß die Reaktion in Gegenwart einer organischen Säure als Stabilisator durchgeführt wird.

6. Verfahren nach Anspruch 5, dadurch gekennzeichnet, daß als organische Säure Zitronensäure, Ascorbinsäure, Weinsäure, Fumarsäure, Oxalsäure oder Äpfelsäure verwendet wird.

## Claims

1. Process for preparing high-purity calcium pyruvate or its hydrates by reacting an aqueous solution of pyruvic acid with an inorganic calcium compound with the pH being set to 2 to 7, preferably 2.5 to 4, characterized in that an up to 10% strength aqueous solution of pyruvic acid is reacted with the calcium compound, in the presence or absence of a stabilizer or complexing agent, and the resulting solution is mixed with an organic diluent at temperatures of 0 to 30°C and the resulting precipitate of calcium pyruvate or its hydrate is isolated from the solution.

2. Process according to Claim 1, characterized in that a 1 to 8% strength aqueous solution of pyruvic acid is used.

3. Process according to Claim 1, characterized in that the reaction is carried out in the presence of a complexing agent for masking impurities due to heavy metals traces.

4. Process according to Claim 3, characterized in that the complexing agent used is ethylenediaminetetraacetic acid or its salts.

5. Process according to Claim 1, characterized in that the reaction is carried out in the presence of an organic acid as stabilizer.

6. Process according to Claim 5, characterized in that the organic acid used is citric acid, ascorbic acid, tartaric acid, fumaric acid, oxalic acid or malic acid.

## Revendications

1. Procédé de préparation de pyruvate de calcium très pur ou de ses hydrates par réaction d'une solution aqueuse d'acide pyruvique avec un composé inorganique du calcium et avec ajustement du pH entre 2 et 7, de préférence entre 2,5 et 4, caractérisé en ce qu'on fait réagir une solution aqueuse d'acide pyruvique à une concentration allant jusqu'à 10 %, éventuellement en présence d'un stabilisant ou d'un agent complexant, avec le composé du calcium, on mélange la solution ainsi obtenue, à des températures de 0 à 30°C, avec un diluant organique et on isole de la solution le précipité formé de pyruvate de calcium ou de ses hydrates.

2. Procédé selon la revendication 1, caractérisé en ce qu'on utilise une solution aqueuse d'acide pyruvique à une concentration de 1 à 8 %.

3. Procédé selon la revendication 1, caractérisé en ce qu'on effectue la réaction en présence d'un agent complexant pour masquer des impuretés produites par des traces de métaux lourds.

4. Procédé selon la revendication 3, caractérisé en ce qu'on utilise de l'acide éthylènediaminetétraacétique ou des sels de celui-ci en tant qu'agent complexant.

5. Procédé selon la revendication 1, caractérisé en ce qu'on effectue la réaction en présence d'un acide organique en tant que stabilisant.

6. Procédé selon la revendication 5, caractérisé en ce qu'on utilise de l'acide citrique, de l'acide ascorbique, de l'acide tartrique, de l'acide fumarique, de l'acide oxalique ou de l'acide malique en tant qu'acide organique.
